# EUROPEAN PATENT APPLICATION

(11) **EP 4 834 869 A1**
(43) Date of publication of application: **04.11.2026**
(21) Application number: 24912570.9
(22) Date of filing: 18.12.2024
(51) Int. Cl.: C07C 4/22, C07B 61/00, C07C 11/04, C07C 11/06, C08J 11/16

(54) **METHOD FOR PRODUCING HYDROCARBON-CONTAINING COMPOSITION**

(30) Priority: 27.12.2023 JP 2023221739
(71) Applicant: Resonac Corporation, Tokyo 105-7325 (JP)
(72) Inventor: FUJITA, Toshio, Tokyo 105-7325 (JP)
(74) Representative: Hoffmann Eitle
(86) International application number: PCT/JP2024/044768
(87) International publication number: WO 2025/142681

(57) **Abstract**

A method for producing a hydrocarbon-containing composition, for producing a hydrocarbon-containing composition containing ethylene, includes a heating step of heating a raw material containing at least one selected from the group consisting of a plastic containing a polyolefin, and a decomposed product thereof, and a catalyst containing 30% by mass to 100% by mass of lanthanum oxide under an inert gas atmosphere while bringing the raw material and the catalyst into contact.

## Description

### TECHNICAL FIELD

The present disclosure relates to a method for producing a hydrocarbon-containing composition.

### BACKGROUND ART

Conventionally, waste plastics have been treated by landfilling, incineration, and the like. However, it is becoming difficult to secure landfill sites, and landfill plastics flow into the oceans and remain as microplastics without being decomposed, causing environmental problems. Furthermore, although waste plastics can be used as heat through incineration, there is a problem that carbon dioxide is emitted, leading to global warming.

In recent years, with increased awareness of the environmental problems, recycling, such as reuse, regeneration, and the like, of waste plastics is required, and research and development for this purpose has been actively conducted. In addition, since most plastics are made from fossil fuels, it is strongly demanded to develop recycling methods from the viewpoint of effective use of the resources.

Among plastic materials, hydrocarbon-based plastic materials, such as polyethylene (PE), polypropylene (PP), polystyrene (PS), and the like, are widely used for beverage and food containers, packaging materials, moldings, films, and the like, because of their excellent properties, such as heat resistance, weather resistance, mechanical strength, transparency, chemical resistance, gas barrier properties, and the like. Therefore, these hydrocarbon-based plastic materials account for the majority of plastic materials, from which waste plastic materials are produced.

One method for recycling these plastic materials is chemical recycling by pyrolysis-gasification. In the pyrolysis-gasification process, waste plastics are converted into low-molecular-weight hydrocarbons by pyrolysis. Here, in order to improve reactivity and selectivity of products, many approaches using solid catalysts have been studied.

For example, production of low-carbon olefins and aromatic hydrocarbons by application of a catalyst to decomposition of waste plastics has been proposed, wherein the catalyst contains: a heat medium; and a catalyst containing a first active component, a second active component, and a third active component, wherein the heat medium contains an oxide of at least one element selected from Si, Al, alkali metals, and alkaline earth metals; the first active component contains an oxide of at least one element selected from Zn, Cu, Ni, Mn, La, Ce, and Ti; the second active component is selected from oxides of alkali metals and/or oxides of alkaline earth metals; and the third active component is selected from oxides of at least one element selected from Zn, Ga, P, Cr, Ag, and K (see PTL 1).

### CITATION LIST

### PATENT LITERATURE

PTL 1: Chinese Patent Publication No. 112316986

### SUMMARY OF THE INVENTION

### TECHNICAL PROBLEM

However, the catalyst disclosed in the prior-art document has a problem that the selectivity of ethylene is low.

An object of the present disclosure is to provide a method for producing a hydrocarbon-containing composition having a high selectivity of ethylene and a good C2-and-C3 O/P ratio.

### SOLUTION TO THE PROBLEM

Means for solving the problems are as follows.
<1> A method for producing a hydrocarbon-containing composition, for producing a hydrocarbon-containing composition containing ethylene, the method including:
   a heating step of heating a raw material containing at least one selected from a group consisting of: a plastic containing a polyolefin; and a decomposed product thereof, and a catalyst containing 30% by mass to 100% by mass of lanthanum oxide, under an inert gas atmosphere, while bringing the raw material and the catalyst into contact.
<2> The method for producing a hydrocarbon-containing composition according to <1>,
   wherein the catalyst further contains an oxide of an alkaline earth metal.
<3> The method for producing a hydrocarbon-containing composition according to <2>,
   wherein a mass ratio of the lanthanum oxide to the oxide of the alkaline earth metal [lanthanum oxide : oxide of alkaline earth metal] in the catalyst is 30:70 to 99:1.
<4> The method for producing a hydrocarbon-containing composition according to <2> or <3>,
   wherein the oxide of the alkaline earth metal is magnesium oxide.
<5> The method for producing a hydrocarbon-containing composition according to any one of <1> to <4>,
   wherein the heating step is heating of (A) the raw material containing at least one selected from the group consisting of: the plastic; and the decomposed product thereof, and (B) a mixture of the catalyst and heat medium particles, while bringing the raw material and the mixture into contact, and
   a mass ratio of the catalyst to the heat medium particles [catalyst : heat medium particles] in the mixture is 1:1 to 1:100.
<6> The method for producing a hydrocarbon-containing composition according to any one of <1> to <5>,
   wherein the plastic contains 50% by mass to 80% by mass of the polyolefin.
<7> The method for producing a hydrocarbon-containing composition according to any one of <1> to <6>,
   wherein the heating step is performed at 300°C to 800°C.

### ADVANTAGEOUS EFFECTS OF THE INVENTION

According to an embodiment of the present disclosure, it is possible to provide a method for producing a hydrocarbon-containing composition having a high selectivity of ethylene and a good C2-and-C3 O/P ratio.

### DETAILED DESCRIPTION OF THE INVENTION

Hereinafter, embodiments of the present disclosure will be described in detail. It should be noted that the embodiments are not intended to be limited by the following description, and may be modified appropriately without departing from the spirit of the present disclosure. In the present specification, the term "to" indicating a numerical range means that the numerical values before and after the term are included as the lower and upper limits unless otherwise specified.

### (Method for Producing Hydrocarbon-Containing Composition)

A method for producing a hydrocarbon-containing composition according to an embodiment, which is a method for producing a hydrocarbon-containing composition containing ethylene, includes a heating step of heating a raw material containing at least one selected from the group consisting of a plastic containing a polyolefin, and a decomposed product thereof (hereinafter may be referred to as "raw material plastic"), and a catalyst containing 30% by mass to 100% by mass of lanthanum oxide under an inert gas atmosphere while bringing the raw material and the catalyst into contact, and further includes other steps as necessary.

The inventor has conducted diligent studies and found that the composition ratio of olefins in hydrocarbon-containing compositions obtained by decomposing raw material plastics differs depending on the metal oxide type. The inventor has also found that use of a catalyst containing, particularly, lanthanum oxide among metal oxides, by 30% by mass to 100% by mass, not only can suitably decompose raw material plastics, but also provides a high selectivity of ethylene useful for recycling plastic materials, among olefins to be contained in hydrocarbon-containing compositions obtained by decomposing the raw material plastics, and a good C2-and-C3 O/P ratio. Therefore, the method for producing a hydrocarbon-containing composition according to an embodiment is also useful for reducing environmental impact.

### <Heating Step>

The heating step is a step of heating a raw material containing at least one selected from the group consisting of a plastic containing a polyolefin and a decomposed product thereof and a catalyst containing 30% by mass to 100% by mass of lanthanum oxide under an inert gas atmosphere while bringing the raw material and the catalyst into contact. Thus, a hydrocarbon-containing composition containing ethylene is obtained.

The heating step is a step of heating (A) the raw material containing at least one selected from the group consisting of the plastic and the decomposed product thereof and (B) a mixture of the catalyst and heat medium particles while bringing the raw material and the mixture into contact, wherein the mass ratio of the catalyst to the heat medium particles in the mixture [catalyst : heat medium particles] is preferably 1:1 to 1:100.

The heating step is performed under an inert gas atmosphere. The inert gas is not particularly limited and can be suitably selected according to the purpose. Examples of the inert gas include nitrogen gas, water vapor, and argon gas, and the inert gas is preferably one selected from the group consisting of nitrogen gas and water vapor. One of these may be used alone, or two or more of these may be used in combination.

Since lanthanum oxide is a solid, the heating step may be a batch reaction or a continuous reaction. When performing a continuous reaction, a fixed bed reactor may be used, or a fluidized bed reactor may be used. When performing a continuous reaction using a fixed bed reactor, an example method is to place the catalyst containing 30% by mass to 100% by mass of lanthanum oxide as a fixed bed in a reaction tube, and add the raw material plastic onto the catalyst layer while causing an inert gas to flow. When performing a continuous reaction, in order to prevent the catalyst from flowing out, it is preferable to use a particulate catalyst. In order to place the catalyst uniformly in the reactor and stabilize the temperature of the catalyst, it is preferable to use heat medium particles.

The heating temperature in the heating step is not particularly limited and may be suitably selected according to the purpose, yet is preferably 300°C to 1,100°C, more preferably 300°C to 800°C, and still more preferably 350°C to 500°C. When the heating temperature is 300°C to 1,100°C, the yield of ethylene is good.

The heating time in the heating step is not particularly limited and may be suitably selected according to the purpose.

In the heating step, the mass ratio of the raw material plastic to the catalyst is not particularly limited and can be appropriately selected according to the purpose. Yet, in the case of the batch reaction, that is, when feeding the raw material plastic all at once, the amount of the catalyst is preferably 20 parts by mass to 500 parts by mass, more preferably 35 parts by mass to 300 parts by mass, and more preferably 50 parts by mass to 100 parts by mass relative to 100 parts by mass of the raw material plastic. By bringing 20 parts by mass to 500 parts by mass of the catalyst into contact with 100 parts by mass of the raw material plastic, it is possible to suitably produce a hydrocarbon-containing composition having a high selectivity of ethylene and a good C2- and-C3 O/P ratio. In the case of the continuous reaction, that is, when feeding the raw material plastic at intervals or continuously, the amount of the catalyst (layer) is preferably 0.05 parts by mass to 15,000 parts by mass, and more preferably 30 parts by mass to 800 parts by mass relative to 100 parts by mass per minute of the raw material plastic.

As industrial wastes, there may be cases where relatively homogeneous waste plastic materials composed of specific plastic components and having a molecular weight distribution within a certain range are obtained. In these cases, it is preferable to adjust the treatment conditions in the heating step in accordance with the type and the molecular weight of the plastic components constituting the waste plastic materials. When separating inorganic substances from waste plastic materials which are general wastes, a lab-scale separation device may be used, but separation can also be performed at an industrial level by such a method as magnetic separation, electrostatic separation, and the like at various plants, such as a refinery, a plastic material recycling plant, and the like.

A heat source for the heating step is not particularly limited, and external heating and internal heating may be used.

### <<Raw Material Plastic>>

The raw material plastic contains at least one selected from the group consisting of a plastic containing polyolefin and a decomposed product thereof, and further contains other components as necessary.

### -Plastic Containing Polyolefin-

The polyolefin is not particularly limited and can be suitably selected according to the purpose, yet is preferably polyethylene (PE), polypropylene (PP), and the like, which are widely used for beverage and food containers, packaging materials, moldings, films, and the like.

The content ratio of the polyolefin in the plastic containing the polyolefin is not particularly limited, can be suitably selected according to the purpose, yet is preferably 50% by mass to 80% by mass, and more preferably 60% by mass to 80% by mass relative to the total mass of the plastic containing the polyolefin. When the content ratio of the polyolefin in the plastic containing the polyolefin is 50% by mass to 80% by mass, it is possible to not only use easily available mixed plastics without separation, but also suitably obtain a hydrocarbon-containing composition having a high selectivity of light olefins, such as ethylene.

In addition to the polyolefin, the plastic containing the polyolefin may contain other plastics, such as polystyrene (PS), polyethylene terephthalate (PET), polybutylene terephthalate (PBT), polyethylene naphthalate (PEN), polycarbonate (PC), polyvinyl chloride (PVC), polyvinylidene chloride, chlorinated polyethylene, polyamide, polyurethane, acrylonitrile-butadiene-styrene copolymer, polymethyl methacrylate and the like.

The content ratio of plastics other than the polyolefin in the plastic containing the polyolefin is not particularly limited, can be suitably selected in accordance with the type of the plastic to be used and the like, yet in view of the yield of ethylene, is preferably less than 50% by mass, more preferably 45% by mass or less, and still more preferably 40% by mass or less relative to the total mass of the plastic containing the polyolefin.

It is preferable that the plastic containing the polyolefin contains waste plastic from the viewpoint of reducing environmental impact.

When the plastic containing the polyolefin is waste plastic, the composition and the composition ratio are not particularly limited and can be suitably selected in accordance with the purpose. Yet, it is preferable that at least one selected from the group consisting of PE and PP is contained. It is more preferable that 20% by mass to 80% by mass of PE and 20% by mass to 80% by mass of PP are contained. It is still more preferable that 20% by mass to 40% by mass of PE, 20% by mass to 40% by mass of PP, and 10% by mass to 30% by mass of PS are contained.

The state of the plastic containing the polyolefin in the heating step is not particularly limited, and examples of the state include a crystalline state, a glassy state, a rubbery state, a liquid state, and the like.

When the plastic containing the polyolefin is crystalline or glassy, the form thereof is not particularly limited. Examples of the form include a pulverized product of the plastic, a pellet composed of a pulverized product of the plastic, a chip composed of a pulverized product of the plastic, and the like.

The pulverized product of the plastic is not particularly limited and can be suitably selected according to the purpose. Examples of the pulverized product include a powder or a flake.

The rubbery or liquid plastic containing the polyolefin means the plastic that is in a state of having fluidity that appears at a temperature equal to or higher than the melting point of the plastic containing the polyolefin and lower than the thermal decomposition temperature of the plastic containing the polyolefin. The rubbery or liquid plastic containing the polyolefin is also referred to as "the melt of the plastic containing the polyolefin".

As the raw material plastic of these various forms, those that have been treated to have the forms, separately from the method for producing a hydrocarbon-containing composition of one embodiment, may be used. Alternatively, the raw material plastic may be treated in a pretreatment step described later.

The rubbery or liquid plastic containing the polyolefin has the same molecular weight as that of the crystalline or glassy plastic containing the polyolefin having the same composition. Therefore, the plastic containing the polyolefin and the decomposed product of the plastic containing the polyolefin can be distinguished based on the molecular weight. Since the melting point decreases as the molecular weight decreases due to decomposition, they can be distinguished based on the melting temperature in practice. The melting temperature is measured by the method specified in the heat flux differential scanning calorimetry (DSC) method according to Japanese Industrial Standards (JIS) K7121-2012.

The melting temperature of the plastic containing the polyolefin is not particularly limited and can be selected appropriately according to the raw material to be used, yet is preferably 80°C to 200°C, and more preferably 90°C to 190°C.

### -Decomposed Product of Plastic Containing Polyolefin-

The decomposed product of the plastic containing the polyolefin is a material resulting from the molecular weight of the plastic containing the polyolefin being reduced. Yet, it is preferable that the decomposed product of the plastic containing the polyolefin has a molecular weight greater than that of a decomposed product of the raw material plastic in the hydrocarbon-containing composition.

The decomposed product of the plastic containing the polyolefin is not particularly limited, and can be selected appropriately according to the raw material to be used. Yet, from the viewpoint of transportation and storage, a product that is liquid at 25°C is preferable, and a product that has an initial boiling point of 30°C or higher is more preferable. The initial boiling point is measured according to the atmospheric pressure method of JIS K2254:2018.

As the decomposed product of the plastic containing the polyolefin, one that has been decomposed separately from the method for producing a hydrocarbon-containing composition of one embodiment may be used. Alternatively, the plastic containing the polyolefin may be decomposed in a decomposition step described later.

### -Other Components-

The other components in the plastic containing the polyolefin are not particularly limited and may be appropriately selected according to the purpose. Examples of the other components include materials other than plastics, additives, and the like. Examples of the materials other than plastics include materials usually contained in waste plastics and the like. Specific examples of the materials other than plastics include paper, metals, and the like.

The content ratio of the materials other than plastics or additives in the raw material plastic is not particularly limited.

### <<Catalyst>>

The catalyst contains 30% by mass to 100% by mass of lanthanum oxide (La₂O₃) and, if necessary, contains other components.

### -Lanthanum Oxide-

Lanthanum oxide is an inorganic compound containing lanthanum, which is a rare earth element, and oxygen, and is La₂O₃.

The content ratio of lanthanum oxide in the catalyst is 30% by mass to 100% by mass relative to the total mass of the catalyst, yet is preferably 30% by mass to 90% by mass, more preferably 30% by mass to 70% by mass, and still more preferably 40% by mass to 60% by mass. When the content ratio of lanthanum oxide in the catalyst is less than 30% by mass, the selectivity of ethylene decreases, and the C2-and-C3 O/P ratio decreases.

### -Other Components-

The other components in the catalyst are not particularly limited and can be selected appropriately according to the purpose. It is preferable that the catalyst further contains an oxide of an alkaline earth metal. The catalyst may also contain a caking additive (binder), a granulation aid, and the like as necessary.

The oxide of an alkaline earth metal among the other components is not particularly limited and can be selected appropriately according to the purpose. Examples include beryllium oxide (BeO), magnesium oxide (MgO), calcium oxide (CaO), strontium oxide (SrO), barium oxide (BaO), radium oxide (RaO), and the like. One of these may be used alone, or two or more of these may be used in combination. Among these, magnesium oxide is preferable as the oxide of an alkaline earth metal because it can improve the C2-and-C3 O/P ratio.

The content of the oxide of an alkaline earth metal in the catalyst is not particularly limited, can be selected appropriately according to the purpose, yet is preferably 30% by mass to 70% by mass, more preferably 40% by mass to 65% by mass, and still more preferably 40% by mass to 60% by mass relative to the total mass of the catalyst.

When the catalyst contains an oxide of an alkaline earth metal, the mass ratio of lanthanum oxide to the oxide of an alkaline earth metal [lanthanum oxide : oxide of alkaline earth metal] in the catalyst is not particularly limited and can be selected appropriately according to the purpose, yet is preferably 30:70 to 99:1, more preferably 30:70 to 70:30, and still more preferably 40:60 to 60:40.

### <<Heat Medium Particles>>

It is preferable to use the heat medium particles in order to maintain the temperature of the reaction system at a constant temperature in the heating step.

The heat medium particles are not particularly limited and can be appropriately selected according to the purpose. It is preferable that the heat medium particles have sufficient heat resistance and chemical stability in the heating step. Specific examples of the heat medium particles include silicon carbide, iron, alumina, zirconia, silica, ceramics, glass, carbon, boron carbide, and the like. One of these may be used alone, or two or more of these may be used in combination. Among these, it is preferable the heat medium particles are silicon carbide. The heat medium particles may be those obtained by surface treatment of the above-mentioned materials for the purpose of deactivating the surface, improving fluidization of plastic melts and decomposed products, and the like.

The size of the heat medium particles is not particularly limited and may be suitably selected according to the purpose. Yet, the size, expressed as the nominal mesh size, is preferably 4.75 mm to 275 mm, and more preferably 2.36 mm to 38 mm. The size of the heat medium particles is measured in accordance with JIS Z 8801-1:2019.

The shape and structure of the heat medium particles are not particularly limited and may be suitably selected according to the purpose.

In the heating step, it is preferable to use the heat medium particles in the form of a mixture of the catalyst and the heat medium particles. The mass ratio of the catalyst and the heat medium particles [catalyst : heat medium particles] in the mixture is not particularly limited and may be suitably selected according to the purpose, yet is preferably 1:1 to 1:100, more preferably 1:1 to 1:50, still more preferably 1:1 to 1:20, and particularly preferably 1:1 to 1:10. When the mass ratio [catalyst : heat medium particles] is 1:1 or greater, the heating temperature tends to be maintained. When the mass ratio [catalyst : heat medium particles] is 1:100 or less, the catalyst and the raw material plastic can be suitably brought into contact.

### <Hydrocarbon-Containing Composition>

The hydrocarbon-containing composition obtained by the method for producing a hydrocarbon-containing composition according to one embodiment contains at least ethylene, and preferably further contains a C3 to C5 olefin. There is a case where the hydrocarbon-containing composition obtained by the method for producing a hydrocarbon-containing composition according to one embodiment contains sub-components other than light olefins.

### -Ethylene-

Ethylene is a C2 olefin.

The content ratio of ethylene in the hydrocarbon-containing composition is not particularly limited and can be suitably selected according to the purpose, yet is preferably 20mol% or greater, more preferably 22mol% or greater, still more preferably 24mol% or greater, and particularly preferably 27mol% or greater relative to the total mole number of carbon in the hydrocarbon-containing composition. The upper limit of the content ratio of ethylene in the hydrocarbon-containing composition is not particularly limited because a higher content ratio is better, yet is preferably 50mol% or less, more preferably 40mol% or less, and still more preferably 35mol% or less. The lower limit and the upper limit of the content ratio of ethylene in the hydrocarbon-containing composition can be suitably combined, and the content ratio of ethylene is preferably 20mol% to 50mol%, more preferably 22mol% to 40mol%, still more preferably 24mol% to 40mol%, and particularly preferably 27mol% to 35mol%.

### -C3 to C5 Olefin-

It is preferable that the C3 to C5 olefin is at least one selected from the group consisting of C3 to C5 alkenes and C3 to C5 dienes, and is more preferably a C3 to C5 alkene.

Examples of the C3 olefin include propylene. The method for producing a hydrocarbon-containing composition according to one embodiment is advantageous not only in high selectivity of ethylene but also in high selectivity of propylene.

Examples of the C4 olefin include 1-butene, cis-2-butene, trans-2-butene, 2-methylpropene, and the like.

Examples of the C5 olefin include 1-pentene, cis-2-pentene, trans-2-pentene, 2-methyl-1-butene, 2-methyl-2-butene, and the like.

The total content ratio of ethylene and propylene in the hydrocarbon-containing composition is not particularly limited and can be suitably selected according to the purpose, yet is preferably 50mol% to 80mol%, and more preferably 50mol% to 70mol% relative to the total mole number of carbon in the hydrocarbon-containing composition.

The total content ratio of C2 to C5 olefins in the hydrocarbon-containing composition is not particularly limited and can be suitably selected according to the purpose, yet is preferably 50mol% to 90mol%, and more preferably 60mol% to 80mol% relative to the total mole number of carbon in the hydrocarbon-containing composition.

In the present disclosure, C2 to C5 olefins encompassing ethylene may be referred to as "light olefins" or "effective components".

The light olefins are raw materials of polyolefins, and polyolefins can be suitably used as raw materials in various fields, such as plastic bags, plastic wraps, drinking straws, medical devices, housings of household electric appliances, erasers, hoses, tires, tubes, CD cases, food trays, food containers, plastic bottles, fibers, and the like.

### -Sub-components-

Examples of the sub-components include an aromatic compound, paraffin, and the like.

### --Aromatic Compound--

The aromatic compound is not particularly limited, yet is preferably benzene, toluene, ethylbenzene, three positional isomers of xylene (p-xylene, m-xylene, and o-xylene), styrene, and cumene.

The content ratio of the aromatic compound in the hydrocarbon-containing composition is not particularly limited and can be suitably selected according to the purpose.

Aromatic compounds are raw materials for resins, and resins can be suitably used as raw materials in various fields, such as CD cases, food trays, food containers, PET bottles, fibers, and the like.

### --Paraffin--

The paraffin is not particularly limited, yet is preferably a C2 to C5 aliphatic saturated hydrocarbon, more preferably a C2 to C5 linear aliphatic saturated hydrocarbon, and still more preferably a C2 and C3 linear aliphatic saturated hydrocarbon. Specific examples of the paraffin include methane, ethane, propane, iso-butane, n-butane, n-pentane, and the like. The method for producing a hydrocarbon-containing composition according to one embodiment has a low selectivity of methane among these.

The lower the content ratio of methane in the hydrocarbon-containing composition, the better. Yet, it is more preferably 20mol% or less, still more preferably 15mol% or less, and particularly preferably 10mol% or less relative to the total mole number of carbon in the hydrocarbon-containing composition.

The total content ratio of ethane and propane in the hydrocarbon-containing composition is preferably 30mol% or less, more preferably 25mol% or less, and still more preferably 20mol% or less relative to the total mole number of carbon in the hydrocarbon-containing composition.

The total content ratio of C2 to C5 paraffins in the hydrocarbon-containing composition is preferably 35mol% or less, more preferably 30mol% or less, and still more preferably 25mol% or less relative to the total mole number of carbon in the hydrocarbon-containing composition.

### --O/P Ratio--

In the present disclosure, it is possible to evaluate a low content of sub-component paraffins, by determining the ratio of the total content ratio (%) of olefins in the hydrocarbon-containing composition to the total content ratio (%) of paraffins in the hydrocarbon-containing composition, that is, the ratio [total content ratio (%) of olefins/total content ratio (%) of paraffins] (hereinafter, may be referred to as "O/P ratio"). The ratio [total content ratio (%) of olefins/total content ratio (%) of paraffins] is evaluated by a ratio [total content ratio (%) of C2 and C3 olefins/total content ratio (%) of C2 and C3 paraffins] or a ratio [total content ratio (%) of C2 to C5 olefins/total content ratio (%) of C2 to C5 paraffins].

As the O/P ratio, the C2-and-C3 O/P ratio and the C2-to-C5 O/P ratio are both not particularly limited and can be selected appropriately according to the purpose, yet are preferably 1.0 or greater, more preferably 2.0 or greater, and still more preferably 3.0 or greater. The higher the value of the O/P ratio, the better. Therefore, the upper limit of the O/P ratio is not particularly limited, yet is preferably 50 or less, and more preferably 20 or less.

The content ratios of ethylene and other olefins contained in the hydrocarbon-containing composition and the O/P ratio can be determined by analyzing the hydrocarbon-containing composition, more specifically, a gaseous product, obtained in the decomposition step, using a gas chromatograph (GC) device equipped with a hydrogen flame ionization detector under the analysis conditions described in the Examples, and quantifying each component based on the peak area ratio of each component to the internal standard by the internal standard method. The internal standard is not particularly limited as long as it is stable under the analysis conditions and can be easily separated from the component to be analyzed. Examples of the internal standard include cyclopentane. In the present disclosure, the content ratio based on the mole number of carbon may be expressed as "Cmol%".

When analyzing an aromatic compound as an olefin-containing composition obtained in the decomposition step, it is possible to determine the content ratio by separating liquid substances including the aromatic compound and the gaseous product in a recovery step described later by, for example, providing a liquid trap filled with tetrahydrofuran (THF) between the reactor and a gas recovery section, analyzing any product soluble in tetrahydrofuran (THF) (hereinafter may be referred to as "THF soluble product") using the gas chromatograph (GC) device equipped with the hydrogen flame ionization detector under the following analysis conditions, and quantifying each component by the internal standard method based on the peak area ratio of each component to the internal standard. In this case, 1-pentene, cis-2-pentene, trans-2-pentene, 2-methyl-1-butene, and 2-methyl-2-butene, which are C5 olefins, and n-pentane, which is a C2 to C5 linear aliphatic saturated hydrocarbon, are also separated as liquid substances. Therefore, in order to calculate the total content ratio of C2 to C5 olefins and the C2-to-C5 O/P ratio, it is necessary to add the analysis results of the liquid substances and the analysis results of the gaseous product. The internal standard used for the analysis of the liquid substances is not particularly limited as long as it is stable under the analysis conditions and can be easily separated from the component to be analyzed. Examples of the internal standard include t-butylbenzene.

### [GC Analysis Conditions for THF Soluble Products]

- Device: Nexis GC-2030 (available from Shimadzu Corporation)
- Columns: DB-1 (Diameter: 0.25 mm, Length: 30 m, available from Agilent Technologies Inc.)
- Carrier Gas Type: He
- Carrier Gas Flow Rate: 97 mL/min
- Injection Temperature: 350°C
- Sample Injection Volume: 1 µL
- Split Ratio: 1/50
- Column Temperature: the temperature raising program is set in the order of 35°C (for 10 minutes), to temperature raising (at 5°C/minute), and to 350°C (for 10 minutes)
- Detector: Hydrogen Flame Ionization Detector (FID)
- Detector Temperature: 350°C

### <Other Steps>

Other steps in the method for producing a hydrocarbon-containing composition are not particularly limited and can be appropriately selected according to the purpose. Examples of the other steps include a pretreatment step for the raw material plastic, a decomposition step for the raw material plastic, a recovery step for an effective component obtained in the heating step, and a separation step for the effective component.

### <<Pretreatment Step>>

The pretreatment step is a step of pretreating the plastic containing the polyolefin before subjecting it to the heating step. By bringing the plastic containing the polyolefin into a form or state that can be easily decomposed in the pretreatment step, it is possible to decompose the plastic containing the polyolefin more efficiently in the heating step.

Examples of the pretreatment include pulverization of the plastic containing the polyolefin, pelletization (shaping into a chip shape) of the pulverized product of the plastic containing the polyolefin, and melting of the plastic containing the polyolefin.

The method for obtaining the pulverized product of the plastic containing the polyolefin is not particularly limited and can be appropriately selected from conventionally publicly-known methods. Examples of the methods include a method of obtaining powder or flakes by pulverizing the plastic containing the polyolefin with a pulverizer.

The method for pelletizing (shaping into a chip shape) the pulverized product is also not particularly limited and can be appropriately selected from conventionally publicly-known methods. Examples of the methods include a method of obtaining a chip-shaped raw material by melt-extruding the pulverized product and then cutting a strand-shaped melt extrudate.

The melting of the plastic containing the polyolefin is not particularly limited and can be suitably selected from conventionally publicly-known method. Yet, it is preferable to treat the plastic containing the polyolefin at a temperature equal to or higher than the melting point of the plastic containing the polyolefin and lower than the thermal decomposition temperature of the plastic containing the polyolefin. It is more preferable to treat it at 100°C to 300°C.

The plastic containing the polyolefin can also be supplied to the heating step in the form of a melt. Examples include a method of continuously supplying the plastic to the heating step using a melt extruder.

### <<Decomposition Step>>

The decomposition step is a step of decomposing the plastic containing the polyolefin before being subjected to the heating step. In the decomposition step, the plastic containing the polyolefin may be used in the raw material state or a pretreated product thereof obtained in a pretreatment step may be used.

The method for decomposing the plastic containing the polyolefin is not particularly limited and may be suitably selected from conventionally publicly-known methods. When decomposing the plastic containing the polyolefin in the absence of the catalyst according to the present disclosure, it is preferable to perform this treatment at 300°C to 500°C. When decomposing the plastic containing the polyolefin in the presence of the catalyst according to the present disclosure, it is preferable to perform this treatment at a temperature of 200°C or higher and lower than 300°C.

The plastic containing the polyolefin can also be supplied to the heating step in the form of a decomposed product. Examples include a method of continuously supplying the plastic to the heating step using a melt extruder.

### <<Recovery Step>>

The recovery step is a step of recovering a gas, which is a product containing ethylene and other light olefins obtained in the heating step, and further liquid substances if necessary. The recovery method is not particularly limited and can be appropriately selected from publicly-known methods in accordance with the type of the product obtained. Examples of the recovery method include a method of separating a gaseous product by pressure distillation at normal pressure and separating liquid hydrocarbons by vacuum distillation at normal pressure.

### <<Separation Step>>

The separation step is a step of separating only effective components from the gaseous and liquid substances recovered in the recovery step and removing unnecessary components.

The method for producing a hydrocarbon-containing composition according to one embodiment may produce not only effective components, such as ethylene and other light olefins, and the like, but also C2 to C5 paraffins, which are sub-components.

The method for separating effective components from sub-components in the separation step is not particularly limited, and may be appropriately selected from publicly-known methods in accordance with the types of the products obtained or the types of the sub-components.

Because the method for producing a hydrocarbon-containing composition according to an embodiment described above provides a high selectivity of ethylene and a good C2-and-C3 O/P ratio, a raw material suitable for chemical recycling can be obtained.

### Examples

The present disclosure will be specifically described below by way of Examples and Comparative Examples. However, the present disclosure should not be construed as being limited to these Examples and Comparative Examples.

### (Example 1)

One gram of lanthanum oxide (obtained from Kanto Chemical Co., Inc.) and 4 g of silicon carbide (Carborundum C80, obtained from Nichika Inc.) were weighed out and mixed by being sufficiently stirred using a stirring bar, to obtain a mixture of a catalyst and heat medium particles. Separately, a plastic composition was prepared by mixing polyethylene (HI-ZEX (registered trademark) 1300 J, obtained from Prime Polymer Co., Ltd.), polypropylene (PRIME POLYPRO (registered trademark) J108M, obtained from Prime Polymer Co., Ltd.), and polystyrene (PSJ-Polystyrene GPPS SGP-10, obtained from PS Japan Corporation) at 1:1:1 (w/w). A quartz test tube having an inner diameter of 18 mm was charged with the total amount of 5 g of the mixture of the catalyst and the heat medium particles, and then with 2.1 g of the mixed plastic composition, and then capped. A gas bag was connected to an outlet of the cap of the quartz test tube. Next, the quartz test tube was set in a tubular furnace (ARF-40KC, obtained from ASH), to heat the materials at 450°C under a nitrogen stream of 15 mL/min and trap a generated gas in the gas bag for 10 minutes.

### (Example 2)

Gas trapping was performed in the same manner as in Example 1 except that 1 g of lanthanum oxide as the catalyst in Example 1 was changed to a mixture of 0.5 g of lanthanum oxide (obtained from Kanto Chemical Co., Inc.) and 0.5 g of magnesium oxide (SAJ special grade, obtained from Sigma-Aldrich Co. LLC).

### (Comparative Example 1)

Gas trapping was performed in the same manner as in Example 1, except that 1 g of lanthanum oxide as the catalyst in Example 1 was changed to 1 g of nickel (II) oxide (reagent special grade, obtained from Kanto Chemical Co., Inc.).

### (Comparative Example 2)

Gas trapping was performed in the same manner as in Example 1, except that 1 g of lanthanum oxide as the catalyst in Example 1 was changed to 1 g of cerium oxide (>99.0%, obtained from Sigma-Aldrich Co. LLC).

### (Comparative Example 3)

Gas trapping was performed in the same manner as in Example 1, except that 1 g of lanthanum oxide as the catalyst in Example 1 was changed to 1 g of magnesium oxide (SAJ special grade, obtained from Sigma-Aldrich Co. LLC).

### <Analysis of Gas Bag Contents>

The proportions of effective components and sub-components in the pyrolyzed gas trapped in the gas bag in Examples 1 and 2 and Comparative Examples 1 to 3 were determined as the mole numbers of carbon (Cmol%) by the following method. Specifically, 40 µL of cyclopentane (>98.0%, obtained from Tokyo Chemical Industry Co., Ltd.) was added to each of the gas bags of Examples 1 and 2 and Comparative Examples 1 to 3, respectively. The gas bags were heated to approximately 40°C to completely evaporate the contents, and then the gas bags were lightly rubbed to mix the contents. The obtained contents were analyzed by the gas chromatograph (GC) method under the following GC analysis conditions, and the proportion (Cmol%) of each component in the pyrolyzed gas in the gas bag was determined based on the ratio of the peak area of each component to the peak area of cyclopentane. The results are shown in Table 1 below.

### [GC Analysis Conditions]

- Device: Nexis GC-2030 (obtained from Shimadzu Corporation)
- Columns: Rt-Aluminum BOND (Diameter: 0.32 mm, Length: 30 m, obtained from Restek Corporation)
- Carrier Gas Type: Ar
- Carrier Gas Flow Rate: 360 mL/min
- Injection Temperature: 200°C
- Sample Injection Volume: 1 mL
- Split Ratio: 1/200
- Column Temperature: After retention at 120°C for 9 minutes, the temperature was raised to 200°C at 10°C/minute, and retained at 200°C for 30 minutes.
- Detector: Hydrogen Flame Ionization Detector (FID)
- Detector Temperature: 200°C

**Table 1**

| | | | **Ex. 1** | **Ex. 2** | **Comp. Ex. 1** | **Comp. Ex. 2** | **Comp. Ex. 3** |
|---|---|---|---|---|---|---|---|
| **Catalyst (g)** | **La₂O₃** | | **1.0** | **0.5** | **-** | **-** | **-** |
| | **MgO** | | **-** | **0.5** | **-** | **-** | **1.0** |
| | **NiO** | | **-** | **-** | **1.0** | **-** | **-** |
| | **CeO₂** | | **-** | **-** | **-** | **1.0** | **-** |
| **Pyrolyzed gas composition (Cmol%)** | **Effective component** | **Ethylene** | **29.7** | **24.2** | **10.8** | **12.4** | **13.5** |
| | | **Propylene** | **31.6** | **43.9** | **16.0** | **28.8** | **45.8** |
| | | **Total of ethylene and propylene** | **61.3** | **68.1** | **26.8** | **41.2** | **59.3** |
| | | **Total of C2 to C5 olefins** | **64.9** | **75.2** | **41.5** | **51.2** | **63.1** |
| | **Sub-component** | **Methane** | **12.1** | **5.5** | **45.1** | **21.3** | **10.1** |
| | | **Total of C2 and C3 paraffins** | **19.7** | **17.3** | **9.6** | **24.2** | **24.5** |
| | | **Total of C2 to C5 paraffins** | **20.9** | **19.1** | **12.5** | **26.7** | **26.5** |
| | **C2-and-C3 O/P ratio** | | **3.1** | **3.9** | **2.8** | **1.7** | **2.4** |
| | **C2-to-C5 O/P ratio** | | **3.1** | **3.9** | **3.3** | **1.9** | **2.4** |

Examples 1 and 2 resulted in higher yields of ethylene, which was an effective component, and a better C2-and-C3 O/P ratio than in Comparative Examples 1 to 3.

As above, the present disclosure has been described based on a specific embodiment and Examples. However, the embodiment and Examples are provided by way of example only, and the present disclosure is not limited by the above embodiment and Examples. The above embodiment can be carried out in various other forms, and various combinations, omissions, substitutions, additions, modifications, and the like are applicable without departing from the spirit of the invention. The embodiment and modifications thereof are included in the scope and spirit of the invention, and are included in the scope of the invention described in the claims and equivalents thereof.

This international application claims priority to Japanese Patent Application No. 2023-221739, filed December 27, 2023, and the entire contents of Japanese Patent Application No. 2023-221739 are incorporated in this international publication by reference.

## Claims

1. A method for producing a hydrocarbon-containing composition, for producing a hydrocarbon-containing composition containing ethylene, the method comprising:
a heating step of heating a raw material containing at least one selected from a group consisting of: a plastic containing a polyolefin; and a decomposed product thereof, and a catalyst containing 30% by mass to 100% by mass of lanthanum oxide, under an inert gas atmosphere, while bringing the raw material and the catalyst into contact.

2. The method for producing a hydrocarbon-containing composition according to claim 1,
wherein the catalyst further contains an oxide of an alkaline earth metal.

3. The method for producing a hydrocarbon-containing composition according to claim 2,
wherein a mass ratio of the lanthanum oxide to the oxide of the alkaline earth metal [lanthanum oxide : oxide of alkaline earth metal] in the catalyst is 30:70 to 99:1.

4. The method for producing a hydrocarbon-containing composition according to claim 2 or 3,
wherein the oxide of the alkaline earth metal is magnesium oxide.

5. The method for producing a hydrocarbon-containing composition according to any one of claims 1 to 4,
wherein the heating step is heating of (A) the raw material containing at least one selected from the group consisting of: the plastic; and the decomposed product thereof, and (B) a mixture of the catalyst and heat medium particles, while bringing the raw material and the mixture into contact, and
a mass ratio of the catalyst to the heat medium particles [catalyst : heat medium particles] in the mixture is 1:1 to 1:100.

6. The method for producing a hydrocarbon-containing composition according to any one of claims 1 to 5,
wherein the plastic contains 50% by mass to 80% by mass of the polyolefin.

7. The method for producing a hydrocarbon-containing composition according to any one of claims 1 to 6,
wherein the heating step is performed at 300°C to 800°C.
